(19) Europäisches Patentamt
European Patent Office
Office européen des brevets

(11) Veröffentlichungsnummer: **0 014 479**
**B1**

(12) **EUROPÄISCHE PATENTSCHRIFT**

(45) Veröffentlichungstag der Patentschrift: **29.06.83**

(51) Int. Cl.$^3$: **A 61 K 7/06**, A 61 K 7/09

(21) Anmeldenummer: **80100689.1**

(22) Anmeldetag: **11.02.80**

(54) **Haarbehandlungsmittel.**

(30) Priorität: **12.02.79 DE 2905257**

(43) Veröffentlichungstag der Anmeldung:
**20.08.80 Patentblatt 80/17**

(45) Bekanntmachung des Hinweises auf die Patenterteilung:
**29.06.83 Patentblatt 83/26**

(84) Benannte Vertragsstaaten:
**BE DE FR GB IT NL SE**

(56) Entgegenhaltungen:
**BE - A - 839 646**
**FR - A - 1 588 952**
**GB - A - 2 025 228**
**US - A - 3 876 760**

(73) Patentinhaber: **Wella Aktiengesellschaft**
**Berliner Allee 65**
**D-6100 Darmstadt (DE)**

(72) Erfinder: **Wajaroff, Theodor**
**Erbacher Strasse 56 b**
**D-6100 Darmstadt (DE)**

**EP 0 014 479 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Haarbehandlungsmittel

Die vorliegende Erfindung betrifft Mittel zur Behandlung von Haaren vor dem Wickeln auf Dauerwellwickler.

Es sind bereits Reinigungsmittel bekannt, die eine Kombination von (1) einer organischen oberflächenaktiven Substanz wie z.B. einem amphoteren kapillaraktiven Imidazolinderivat, (2) mindestens einem wasserlöslichen kationischen, Stickstoff enthaltenden Polymer wie beispielsweise einem quaternären Zellulosederivat oder quaternären Polydimethyl- bzw. Polydiäthylaminoäthylmethacrylat, mit einem Molekulargewicht von 2000—3 000 000 und einer kationischen Ladungsdichte über 0,001 in wäßriger Lösung sowie (3) einer wasser-unlöslichen bzw. schwerlöslichen, teilchenförmigen Substanz wie z.B. Silicon "F—157" der Firma Dow Corning, enthalten. Die Mittel weisen außer der reinigenden Wirkung noch zusätzlich eine spezielle Wirkung auf. Diese wird dadurch erzielt, daß das enthaltene Polymer es ermöglicht, daß nach dem Waschen die ebenfalls enthaltenen teilchenförmigen Substanzen mit ihrer speziellen Wirkung wie Antischuppen-, Desinfektions- oder Sonnenschutzwirkung auf der Haut zurückgehalten werden und dort längere Zeit wirksam bleiben können (vgl. FR—PS 1 588 952).

Weiterhin sind schon Haarbehandlungsmittel bekannt geworden, welche einen Gehalt an verzweigten niedermolekularen Isoparaffinen mit einem Siedebereich von 45°C bis 250°C aufweisen. Die Mittel sind geeignet, die insbesondere bei chemisch vorbehandeltem, wie z.B. dauergewelltem, Haar eintretende Verfilzung der Haare nach dem Waschen zu beseitigen, und das Haar wieder leicht kämmbar und ohne Schwierigkeiten frisierbar zu machen (vgl. BE—PS 839 646).

Schließlich sind bereits Haarfrisiermittel mit einem Gehalt an einem haarsubstantiven quaternären Harz wie z.B. einem quaternisierten Zelluloseäther sowie einer haarpflegenden Substanz wie beispielsweise einem substituierten Imidazolin bekannt. Während das quaternäre Harz imstande ist, auf das Haar aufzuziehen und ihm festen Halt zu geben, soll die haarpflegende Substanz sich auf dem filmartigen Haarzüberzug ablagern und dem Haar pflegende Wirkung wie Geschmeidigkeit, weichen Griff, Glanz, antistatische Eigenschaften, natürliches Aussehen sowie konditionierende Wirkung verleihen (vgl. US—PS 3 876 760).

Gegenstand der Erfindung ist ein Mittel zur Behandlung von Haaren vor dem Wickeln auf Dauerwellwickler.

Beim Dauerwellen der Haare mit reduzierenden Dauerwellmitteln wird heute vorzugsweise so verfahren, daß die zuvor gewaschenen und mit einem Handtuch abfrottierten Haare in Strähnen aufgeteilt werden, diese Strähnen sodann einzeln auf Wickler gewickelt und anschließend mit der reduzierenden Dauerwellösung befeuchtet werden. Dieses Verfahren weist gegenüber der älteren Verfahrensweise, wonach die Haare bereits vor dem Wickeln mit der Dauerwellösung vorgefeuchtet werden, verschiedene Vorteile auf. Zum einen wird hierbei der sonst zum Wickeln der Haare erforderliche, etwa 20 Minuten dauernde, Kontakt der Hände des Friseurs mit der Dauerwellösung vermieden, zum anderen erfolgt die Dauerwellung unter haarschonenderen Bedingungen. Die beim Vorfeuchten des Haares mit der Dauerwellösung eintretende Haarerweichung kann nämlich beim Wickelvorgang zu einer Überdehnung des Haares und später zu Haarbruch und Haarausfall führen.

Das Aufwickeln der einzelnen, parallel gekämmten Haarsträhnen soll unter leichtem Zug erfolgen, wobei der Zug möglichst gleichmäßig auf alle Haare zu verteilen ist. Diesen schwierigen Vorgang versucht man durch Verwendung eines sogenannten "Spitzenpapiers" zu erleichtern. Dabei werden die Haarspitzen zwischen ein gefaltetes dünnes Blatt — vorzugsweise aus Papier — gelegt und dann gewickelt.

Nach der erforderlichen Einwirkungszeit des reduzierenden Dauerwellmittels auf die gewickelten Haare, die je nach der Haarbeschaffenheit und den äußeren Umständen etwa 10 bis 30 Minuten beträgt, wird das Haar gründlich gespült und anschließend durch eine oxidierende Behandlung, beispielsweise mit einer Wasserstoffperoxidlösung, in der gewünschten Form fixiert.

Das Haar hat zwischen Haaransatz und Haarspitze ein unterschiedliches Aufnahmevermögen für die Dauerwellflüssigkeit. So quellen beispielsweise die Haarspitzen am meisten auf und zeigen die stärkste Verformung. Ihre starke Verformung ist ferner auch darauf zurückzuführen, daß die Haarspitzen direkt am Wickler anliegen und, bezogen auf die gesamte Haarsträhne, am stärksten gekrümmt werden.

Die bei der Dauerwellbehandlung nach dem geschilderten Stand der Technik auftretenden Haarschädigungen machen sich unter anderem durch mangelhafte Kämmbarkeit des Haares und ungenügende Haltbarkeit der Haarkrause, insbesondere im Bereich der Haarspitzen, bemerkbar.

Demgegenüber wurde nun gefunden, daß die vorgenannten Nachteile bei der Dauerwellbehandlung von Haaren vermieden werden, wenn man vor dem Wickeln auf Dauerwellwickler das nachstehend beschriebene erfindungsgemäße Mittel auf die Haare aufträgt.

Die erfindungsgemäßen Mittel zur Behandlung von Haaren sind gekennzeichnet durch einen Gehalt an den Komponenten

A) 5 bis 60 Gewichtsprozent mindestens eines Methylpolysiloxans mit einem Siedepunkt im Bereich von 100 bis 190°C und/oder mindestens eines Paraffins bzw. Isoparaffins mit einem Siedepunkt im Bereich von 100 bis 190°C,

B) 0,02 bis 3,0 Gewichtsprozent mindestens einer kationaktiven, nicht kapillaraktiven Verbindung und
C) 0,02 bis 3,0 Gewichtsprozent mindestens einer ampholytischen, kapillaraktiven Imidazolinverbindung, deren Menge die der Komponente B nicht übertreffen soll.

Diese Mittel sollen als Methylpolysiloxan insbesondere die Verbindungen Hexamethyldisiloxan, Octamethyltrisiloxan und Dekamethyltetrasiloxan enthalten. Als Beispiele für in diesen Mitteln enthaltene Paraffine bzw. Isoparaffine sind insbesondere n-Nonan, n-Decan, Methylheptan (Isomerengemisch mit einem Siedepunkt von etwa 118°C), Trimethylpentan (Isomerengemisch mit einem Siedepunkt von 100 bis 114°C) und 2.2.3.3. Tetramethylbutan zu nennen.

Als kationaktive, nicht kapillaraktive Verbindungen, welche in den Mitteln enthalten sein sollen, kommen zum Beispiel a) kationaktive Harze, insbesondere polymeres Dimethylaminoäthylmethacrylat (Homopolymer, zu 75% quaternisiert mit Dimethylsulfat) und polymeres Diallyldimethylammoniumchlorid (Homopolymer) sowie b) kationaktive Cellulose der allgemeinen Formel

$$\left\{ (C_6H_{10}O_5)_n(CH_2{-}CH_2OH)_{n-2n}\left[CH_2{-}\underset{\displaystyle OH}{\underset{|}{CH}}{-}CH_2{-}N(CH_3)_3\right]_{0,2n-0,5n}\right\}^{+}Cl^{-},$$

wobei $n = 500-2500$ ist, in Betracht.

Als ampholytische, kapillaraktive Imidazolinverbindung soll in den Mitteln mindestens ein Imidazolinderivat der allgemeinen Formel

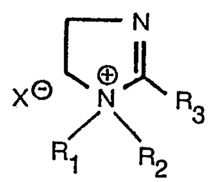

enthalten sein, wobei $X = OH$, Cl, 1/2 $SO_4$, Alkylsulfat wie insbesondere Äthylsulfat, bedeutet und die Reste $R_1$, $R_2$ und $R_3$ die folgenden Gruppen darstellen:

$R_1 = -CH_2-COOA$ (A = H, Na)
$-CH_2-CH_2-OH$

$R_2 = -CH_2-CH_2-O-CH_2-COOA$ (A = H, Na)
$-CH_2-COOA$
$-CH_2-CH_2-OA$

$R_3$ = geradkettiger Kohlenwasserstoffrest mit 7 bis 17 Kohlenstoffatomen, vorzugsweise $C_7H_{15}$, $C_9H_{19}$, $C_{11}H_{23}$ und $C_{17}H_{35}$.

Die Methylsiloxane, Paraffine und Isoparaffine liegen in den erfindungsgemäßen Mitteln vorzugsweise in einer Gesamtkonzentration von zusammen 25 bis 55 Gewichtsprozent vor. Der Gehalt an kationaktiven, nicht kapillaraktiven Verbindungen sowie an ampholytischen, kapillaraktiven Imidazolinverbindungen in den Mitteln Beträgt jeweils vorzugsweise 0,2 bis 3,0 Gewichtsprozent, wobei die Menge der Imidazolinverbindungen die Menge der kationaktiven, nicht kapillaraktiven Verbindungen nicht übertreffen soll.

Die Mittel gemäß vorliegender Erfindung können außerdem in kosmetischen Mitteln gebräuchliche Zusatzstoffe, und zwar Alkohole wie vorzugsweise Äthanol, Propanol und Isopropanol, Lösungsvermittler wie beispielsweise oxäthyliertes Rizinusöl und 4-n-Nonylphenoldekaglykoläther, ferner die Haarquellung fördernde Substanzen wie insbesondere Harnstoff und Alkalisulfite, Alkalisierungsmittel wie Ammoniak, Monoäthanolamin, Ammoniumhydrogencarbonat und Ammoniumcarbonat, weiterhin kapillaraktive, kationaktive Substanzen wie beispielsweise Hexadecyltrimethylammoniumchlorid, Farben, Parfümöle, Glycerin, Lanolin und andere enthalten. Weiterhin kann diesen Mitteln vor ihrem Gebrauch gegebenenfalls ein Ferment, wie beispielsweise Urease, Lipase oder Bierhefe zugegeben werden.

Die hier beschriebenen Haarbehandlungsmittel bestehen aus zwei flüssigen Phasen, von denen die obere Phase die eingangs unter A) angeführten Bestandteile enthält, während sich die eingangs unter B) und C) angeführten Bestandteile in der unteren, wäßrigen Phase befinden.

Die Anwendung der erfindungsgemäßen Haarbehandlungsmittel erfolgt in der Weise, daß die beiden flüssigen Phasen der Mittel unmittelbar von Gebrauch durch kurzes Schütteln in einem geschlossenen Behältnis in eine mehr oder weniger leicht brechende Emulsion übergeführt werden. Diese

wird dann gleichmäßig auf das zuvor gewaschene, handtuchtrokkene Haar aufgetragen. Für eine Haarbehandlung wird hierbei, je nach Haarfülle, eine Menge von etwa 15 bis 25 Gramm des Mittels benötigt. Anschließend werden die Haare durchgekämmt, in Strähnen abgeteilt, sodann auf Wickler gewickelt und schließlich einer üblichen Dauerwellbehandlung unterworfen.

Die vorteilhafte Wirkung der Mittel besteht darin, daß sie das Kämmen, Abteilen in Strähnen sowie das Wickeln der Haare wesentlich erleichtern. Ferner wird die Einwirkung des Dauerwellmittels auf die Haarspitzen herabgesetzt und dadurch ein vom Haaransatz bis zu den Haarspitzen gleichmäßiges Wellergebnis erreicht. Außerdem weist das Haar nach der Dauerwellbehandlung eine ausgezeichnete Haarbeschaffenheit, insbesondere hinsichtlich Griff, Glanz und Kämmbarkeit des Haares, auf.

Die folgenden Beispiele von erfindungsgemäßen Mitteln sind bezüglich der angegebenen Gesamtmengen für jeweils eine Behandlung berechnet.

Durch diese Beispiele soll der Gegenstand der vorliegenden Erfindung näher erläutert werden.

## Beispiele

### Beispiel 1

Mittel, geeignet für alle Haarsorten

| | |
|---|---|
| 10,0 g | Hexamethyldisiloxan |
| 0,3 g | Homopolymer von Dimethylaminoäthylmethacrylat, zu 75% quaternisiert mit Dimethylsulfat |
| 0,1 g | 2-Kokos-1-natriumcarboxymethyl-1-(2-hydroxyäthyl)-imidazoliniumhydroxid |
| 0,3 g | 4-n-Nonylphenoldekaglykoläther |
| 4,0 g | Harnstoff |
| 0,1 g | Parfümöl |
| 10,2 g | Wasser |
| 25,0 g | |

### Beispiel 2

Mittel für normales Haar

| | |
|---|---|
| 8,0 g | Methylheptan, Isomerengemisch mit einem Siedepunkt von etwa 118°C |
| 0,1 g | Kationaktive Cellulose der Formel $\left\{ (C_6H_{10}O_5)_n(CH_2CH_2OH)_{n-2n} \left[CH_2CHOHCH_2N(CH_3)_3\right]_{0,2n-0,5n} \right\}^+Cl^-$ n = 500—2500 |
| 0,1 g | 2-Nonyl-1-natriumcarboxymethyl-1-[2-(natriumcarboxymethoxy)äthyl]-imidazoliniumhydroxid |
| 0,1 g | Rizinusöl, mit 40 Mol Äthylenoxid oxäthyliert |
| 0,2 g | Parfümöl |
| 6,5 g | Wasser |
| 15,0 g | |

### Beispiel 3

Mittel für normales Haar

| | |
|---|---|
| 8,00 g | Gemisch von Isoparaffinen mit einem Siedepunkt von 170 bis 190°C |
| 0,05 g | Copolymerisat von 80% Vinylpyrrolidon und 20% Dimethylaminoäthylmethacrylat (teilweise quaternisiert) |

| | |
|---|---|
| 0,05 g | 2-Heptadecyl-1-carboxymethyl-1-(2-hydroxyäthyl)-imidazoliniumchlorid |
| 0,20 g | Hexadecyltrimethylammoniumchlorid |
| 0,30 g | parfümöl |
| 9,40 g | Wasser |
| 18,00 g | |

Beispiel 4

Mittel für geschädigtes Haar

| | |
|---|---|
| 5,00 g | Octamethyltrisiloxan |
| 0,20 g | Homopolymer von Diallyldimethylammoniumchlorid |
| 0,15 g | Äquimolares Gemisch aus 2-Heptadecyl-1-carboxymethyl-1-(2-hydroxyäthyl)-imidazoliniumchlorid und 2-Heptadecyl-1-di(2-hydroxyäthyl)-imidazoliniumchlorid |
| 3,00 g | Isopropanol |
| 0,10 g | Parfümöl |
| 11,55 g | Wasser |
| 20,00 g | |

Beispiel 5

Mittel für blondiertes Haar

| | |
|---|---|
| 5,0 g | Octamethyltrisiloxan |
| 5,0 g | Dekamethyltetrasiloxan |
| 0,6 g | Homopolymer von Diallyldimethylammoniumchlorid |
| 0,2 g | 2-Undecyl-1-natriumcarboxymethyl-1-[2-(natriumcarboxymethoxy)äthyl]-imidazoliniumhydroxid |
| 0,2 g | Parfümöl |
| 9,0 g | Wasser |
| 20,0 g | |

Alle in der vorliegenden Anmeldung angegebenen Prozentzahlen stellen Gewichtsprozente dar.

**Patentansprüche**

1. Mittel zur Behandlung von Haaren vor dem Wickeln auf Dauerwellwickler, gekennzeichnet durch einen Gehalt an den Komponenten

A) 5 bis 60 Gewichtsprozent mindestens eines Methylpolysiloxans mit einem Siedepunkt im Bereich von 100 bis 190°C und/oder mindestens eines Paraffins bzw. Isoparaffins mit einem Siedepunkt im bereich von 100 bis 190°C,
B) 0,02 bis 3,0 Gewichtsprozent mindestens einer kationaktiven, nicht kapillaraktiven Verbindung und
C) 0,02 bis 3,0 Gewichtsprozent mindestens einer ampholytischen, kapillaraktiven Imidazolinverbindung, deren Menge die der Komponente B nicht übertreffen soll.

2. Mittel nach Anspruch 1, dadurch gekennzeichnet, daß das Methylpolysiloxan das Hexamethyldisiloxan, Octamethyltrisiloxan oder Dekamethyltetrasiloxan ist.

3. Mittel nach Anspruch 1 und 2, dadurch gekennzeichnet, daß das Paraffin n-Nonan oder n-Decan und das Isoparaffin Methylheptan (Isomerengemisch mit einem Siedepunkt von etwa 118°C), Trimethylpentan (Isomerengemisch mit einem Siedepunkt von 100 bis 114°C) oder 2.2.3.3. Tetramethylbutan ist.

4. Mittel nach Anspruch 1 bis 3, dadurch gekennzeichnet, daß die kationaktive, nicht kapillaraktive Verbindung a) ein kationaktives Harz und/oder b) eine kationaktive Cellulose der allgemeinen Formel

$$\left\{ (C_6H_{10}O_5)_n(CH_2\text{—}CH_2OH)_{n-2n} \left[ CH_2\text{—}\underset{\displaystyle OH}{\underset{|}{CH}}\text{—}CH_2\text{—}N(CH_3)_3 \right]_{0{,}2n-0{,}5n} \right\}^{+}Cl^{-},$$

wobei $n = 500$—$2500$ bedeutet ist.

5. Mittel nach Anspruch 1 bis 4, dadurch gekennzeichnet, daß die ampholytische, kapillaraktive Imidazolinverbindung eine Verbindung des Typs

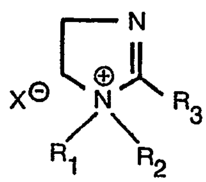

ist, wobei die Reste $R_1$, $R_2$ und $R_3$ folgende Bedeutung haben:

$R_1 =$ —$CH_2$—COOA (A = H, Na)
—$CH_2$—$CH_2$—OH

$R_2 =$ —$CH_2$—$CH_2$—O—$CH_2$—COOA (A = H, Na)
—$CH_2$—COOA
—$CH_2$—$CH_2$—OA

$R_3 =$ geradkettiger Kohlenwasserstoffrest mit 7 bis 17 Kohlenstoffatomen und

X = OH, Cl, 1/2 $SO_4$, Alkylsulfat ist.

6. Mittel nach Anspruch 1 bis 5, dadurch gekennzeichnet, daß es gebräuchliche kosmetische Zusatzstoffe wie niedere Alkohole, Lösungsvermittler, Haarquellmittel, Alkalisierungsmittel, ferner kationaktive, kapillaraktive Substanzen, Farbstoffe, Parfümöle, Glycerin, Lanolin und Fermente enthält.

**Claims**

1. Composition for the treatment of hair before winding it onto permanent-waving rollers, characterised by a content of the components:

A) 5 to 60 weight percent of at least one methylpolysiloxane having a boiling point from 100 to 190°C and/or at least one paraffin or isoparaffin having a boiling point from 100 to 190°C,
B) 0,02 to 3,0 weight percent of at least one cation-active, non-capillary active compound and
C) 0,02 to 3,0 weight percent of at least one ampholytic, capillary-active imidazoline compound, in an amount not exceeding that of component B.

2. Composition according to Claim 1, characterised in that the methyl polysiloxane is hexamethyl disiloxane, octamethyl trisiloxane or decamethyl tetrasiloxane.

3. Composition according to Claim 1 and 2, characterised in that the paraffin is n-nonane or n-decane and the isoparaffin is methyl heptane (a mixture of isomers having a boiling point of about 118°C), trimethyl pentane (a mixture of isomers having a boiling point of 100 to 114°C) or 2,2,3,3 tetramethyl-butane.

4. Composition according to Claim 1 to 3, characterised in that the cation-active, non capillary-active compound (a) is a cation-active resin and/or (b) a cation-active cellulose of the general formula

$$\left\{ (C_6H_{10}O_5)_n(CH_2\text{—}CH_2OH)_{n-2n} \left[ CH_2\text{—}\underset{\displaystyle OH}{\underset{|}{CH}}\text{—}CH_2\text{—}N(CH_3)_3 \right]_{0{,}2n-0{,}5n} \right\}^{+}Cl^{-},$$

in which $n = 500$—$2500$.

5. Composition according to Claim 1 to 4, characterised in that the ampholytic capillary active imidazoline compound is a compound of the type

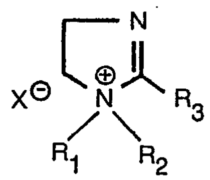

in which the residues $R_1$, $R_2$ and $R_3$ have the following meanings:

$R_1$ = $—CH_2—COOA$ (A = H, Na)
$—CH_2—CH_2—OH$

$R_2$ = $—CH_2—CH_2—O—CH_2—COOA$ (A = H, Na)
$—CH_2—COOA$
$—CH_2—CH_2—OA$

$R_3$ = a straight chain hydrocarbon residue with 7 to 17 carbon atoms and

X = OH, Cl, 1/2 $SO_4$, or alkylsulphate.

6. Composition according to Claim 1 to 5, characterised in that it contains conventional cosmetic additives such as lower alcohols, solubility aids, hair swelling media, alkalisation media, further cation-active, capillary-active substances, dyes, perfume oils, glycerol, lanoline, and enzymes.

**Revendications**

1. Composition pour le traitement des cheveux avant leur enroulement sur des rouleaux à ondulation permanente, caractérisée par une teneur en composants qui est la suivante:

A) entre 5 et 60 pour cent en poids d'au moins un méthylpolysiloxane dont le point d'ébullition est compris dans la plage de 100 à 190°C et/ou au moins une paraffine ou une isoparaffine dont le point d'ébullition est compris dans la plage de 100 à 190°C,
B) de 0,02 à 3,0 pour cent en poids d'au moins un composé cation-actif et non capillaire-actif, et
C) de 0,02 à 3,0 pour cent en poids d'au moins un composé d'imidazoline ampholytique et capillaire-actif, dont la quantité ne doit pas dépasser celle des composants B.

2. Composition selon la revendication 1, caractérisée en ce que le methylpolysiloxane est de l'héxaméthyldisiloxane, de l'octaméthyltrisiloxane ou du décaméthyltétrasiloxane.

3. Composition selon la revendication 1 ou 2, caractérisée en ce que la paraffine est constituée par de l'n-nonane ou du n-décane, et en ce que l'isoparaffine est constituée par le méthylheptane (mélange d'isomères dont le point d'ébullition est d'environ 118°C), le triméthylpentane (mélange d'isomères dont le point d'ébullition est compris entre 100 et 114°C) ou le 2.2.3.3.tétraméthylbutane.

4. Composition selon l'une des revendications 1 à 3, caractérisée en ce que le composé cation-actif et non capillaire-actif est constitué par a) une résine cation-active et/ou b) une cellulose cation-active de formule générale:

$$\left\{ (C_6H_{10}O_5)_n(CH_2—CH_2OH)_{n-2n} \left[ CH_2—\underset{\substack{|\\ OH}}{CH}—CH_2—N(CH_3)_3 \right]_{0,2n-0,5n} \right\}^+ Cl^-,$$

où n = 500—2500.

5. Composition selon l'une des revendications 1 à 4, caractérisée en ce que le composé d'imidazoline capillaire-actif et ampholytique est un composé du type:

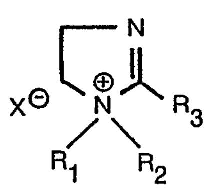

où les radicaux $R_1$, $R_2$ et $R_3$ représentent les groupes suivants:

$R_1 = —CH_2—COOA$ (A = H, Na)
$—CH_2—CH_2—OH$

$R_2 = —CH_2—CH_2—O—CH_2—COOA$ (A = H, Na)
$—CH_2—COOA$
$—CH_2—CH_2—OA$

$R_3$ = radical d'hydrocarbure à chaîne droite comprenant de 7 à 17 atomes de carbone, et

$X^- $ = OH, Cl, 1/2 $SO_4$ ou de l'alcoylsulfate.

6. Composition selon l'une des revendications 1 à 5, caractérisée en ce qu'elle contient des adjuvants cosmétiques habituels tels que des alcools faibles, des médiateurs de solution, des substances qui favorisent le gonflement des cheveux, des agents d'alcalisation, en outre des substances cation-actives, capillaire-actives, des colorants, des huiles parfumées, de la glycérine, de la lanoline et des ferments.